**Europäisches Patentamt**

(19) **European Patent Office**

**Office européen des brevets**

(11) Publication number: **0 289 563 B1**

(12) # EUROPEAN PATENT SPECIFICATION

(45) Date of publication of patent specification:
29.05.91 Bulletin 91/22

(51) Int. Cl.⁵: **A61M 15/00**

(21) Application number: 87907429.2

(22) Date of filing: 05.11.87

(86) International application number:
PCT/FI87/00150

(87) International publication number:
WO 88/03419 19.05.88 Gazette 88/11

(54) INHALATION DEVICE.

(30) Priority: 06.11.86 FI 864505
18.02.87 FI 870678

(43) Date of publication of application:
09.11.88 Bulletin 88/45

(45) Publication of the grant of the patent:
29.05.91 Bulletin 91/22

(84) Designated Contracting States:
AT BE CH DE FR GB IT LI LU NL SE

(56) References cited:
WO-A-10/2982
WO-A-30/3976
SE-B- 344 402
SE-B- 437 935

(73) Proprietor: HUHTAMÄKI OY
Kärsämäentie 35
SF-20100 Turku (FI)

(72) Inventor: LANKINEN, Tapio
Jalustinkatu 11
SF-20800 Turku (FI)
Inventor: LEHTINEN, Matti, Jarmo, Kalevi
Raurolantie
SF-20760 Piispanristi (FI)

(74) Representative: Palgen, Peter, Dr.
Patentanwälte Kuborn & Dr. Palgen
Mulvanystrasse 2
W-4000 Düsseldorf (DE)

## Description

The present invention relates to an inhalation device provided with an inhalation chamber and an inlet duct as well as a mouthpiece for receiving a spray which contains a substance to be inhaled.

The most common form of medicine used in inhalation therapy is an inhalation aerosol. This is an effective form of medicine if used properly, but the correct use is difficult to learn for many patients. It is known that only a small share (circa 10 %) of the medicine reaches the lungs and most of the medicine remains in the mouth, with an added risk of side effects. It has also been stated that less than 20% of the aerosol propellant is gasified upon leaving the nozzle and that the gasification of the propellant takes several seconds.

Various inhalation chambers, into which the aerosol dose is sprayed before inhalation, have been developed in order to overcome the above drawbacks. When sprayed into such a chamber, the propellant droplets have sufficient time to gasify, whereby the medicament particles can be inhaled later. It has been shown that the amount of medicament remaining in mouth and throat is considerably decreasing by using a device with an inhalation chamber but the clinical effect of such a device is not better than a plain inhalation aerosol, if properly used. However, the presently available inhalation chambers are quite bulky, generally about 500-750 ml in capacity, and thus uncomfortable to use and carry along.

Finish Patent publication 64512 (Draco AB, Sweden) discloses an inhalation device the inhalation chamber of which is designed to conform with the conical shape of an aerosol spray generated from an injection nozzle. The size of the chamber is 500-2000 ml. The conical form of the chamber does not improve or support the rotational movement of the aerosol spray in the chamber.

An object of the present invention is to provide an inhalation device, wherein an aerosol spray is set in rotating motion in the inhalation chamber thus improving the gasification of the propellant and liberation of the medicinal particles from the propellant.

A device of the invention is characterized in that the cross-section of an inhalation chamber in a plane defined to the longitudinal axis of the aerosol spray substantially is in the shape of a circle, ellipse, polygon or a combination or interlinked arrays thereof and that the spraying into the chamber is in a non-radial direction in order to achieve a rotational movement of said spray and in that said chamber may be flat at least in one of its lateral directions.

In this invention the form of the chamber is chosen to build up a rotational movement of the aerosol spray. With this new functional principle a more efficient inhalator but smaller in size is obtained. The efficiency is improved with the same or even lower doses of medical aerosol preparations.

The other features of the invention are set forth in the annexed claims.

The function of this new device was studied by photographing the behaviour of a spray sprayed into a round glass bottle (250 ml) (exposure time of 1/1000 seconds). The turbulence was formed right after the triggering action and its fastest speed was circa 60 r/sec in the ball with a 4 cm radius. It was also observed that ungasified propellant drops swirled along with the turbulence and gasified while doing so. The turbulence continued for 3-4 seconds during wich the propellant gasified almost completely.

The construction of a device of the invention was optimized and compared with prior devices by measuring the amount of medicine that could be inhaled. Thus, a dose injected into the chamber was sucked 3 seconds after the triggering action into a particle separator (Andersen 1 AFTM Cascade Impactor) and the thus separated amounts of medicine having different particle sizes were analyzed chromatographically. Each result is the average of 40 sprays. Reference articles were commercially available inhalation chambers : Volumatic R, Nenulator R, Inspri-Ease R, Inhal-Aid R. A device of the invention having the optimal size and shape provided a result at least twice as good as any of the above-mentioned devices when using the same aerosol (Salbubent R). The size of such optimal device was smaller than any of the above-mentioned devices.

The most efficient device proved to be a device with a spherical or spherically compressed inhalation chamber. If the form of the inhalation chamber deviated from a sphere, the rotation velocity and duration decreased as well as the amount of inhalable medicine. If in an ellipsoidal chamber the shortest diameter was less than 80% of the longest diameter an efficient rotation was not obtained.

In a spherical chamber the rotational movement was easily obtained by spraying into the chamber in a non-radial direction, i.e. with slight deviation from the center of the sphere. With increasing deviation (over 10°) the turbulence is slowing down and the inhable product decreases. The best result was obtained when mouthpiece was on the side wall of the chamber perpendicular to the rotational plane of the aerosol spray. The poorest result was obtained when the mouthpiece was on the periphery of the rotation but even then the result were better than the result obtained by the prior art devices. Satisfactory results were also obtained when the inhalation chamber was mate of two or more interconnected spheres or spherelike chambers.

The direction and plane of the rotation are depending on how the axis of the spray is directed to the center of the chamber.

The operation of a device of the invention is very easy. It can be used the same way as a regular aerosol inhalation device, whereby a patient places the mouthpiece in his or her mouth and triggers a dose at the start of inhalation. If this is awkward, a patient can first trigger a dose and then inhale after a while ; the device functions just as effectively. If a one-way valve is employed, the device will be particularly suitable for children and elderly persons whno have most deliciencies in aspiration technique. In this case, it is not possible to exhale through the device, only inhalation is possible.

The invention will now be described in more detail with reference made to the accompanying drawings, in which

Fig. 1 shows a schematic perspective view of an embodiment of the device with cross-section planes a and b ;

Fig. 2 and 3 illustrate routtine of the chamber of a few basic embodiments of a device of the invention in the cross-section planes a and b, respectively ;

Fig. 4-5 show three embodiments of the invention in cross-section and

Fig. 7-12 show still further embodiments of the invention.

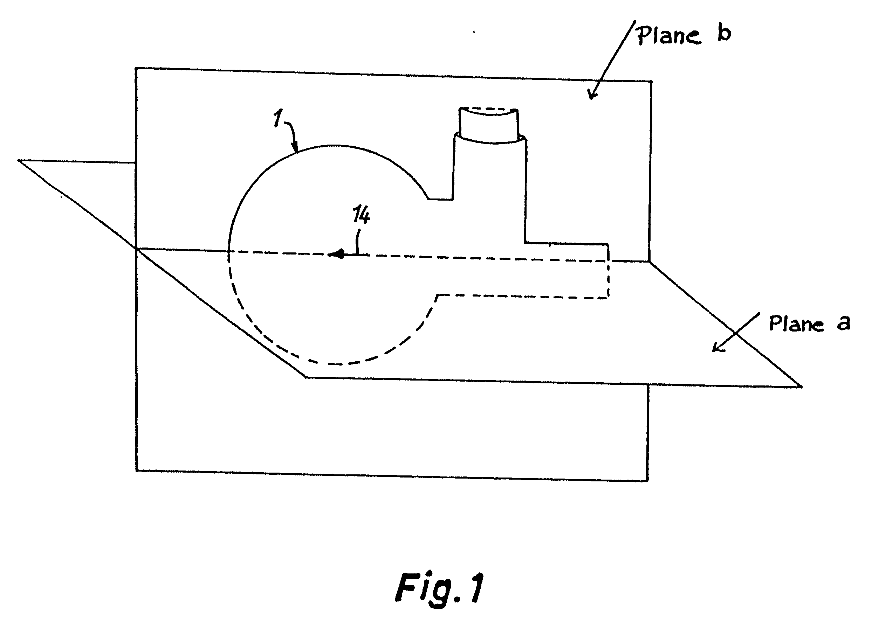

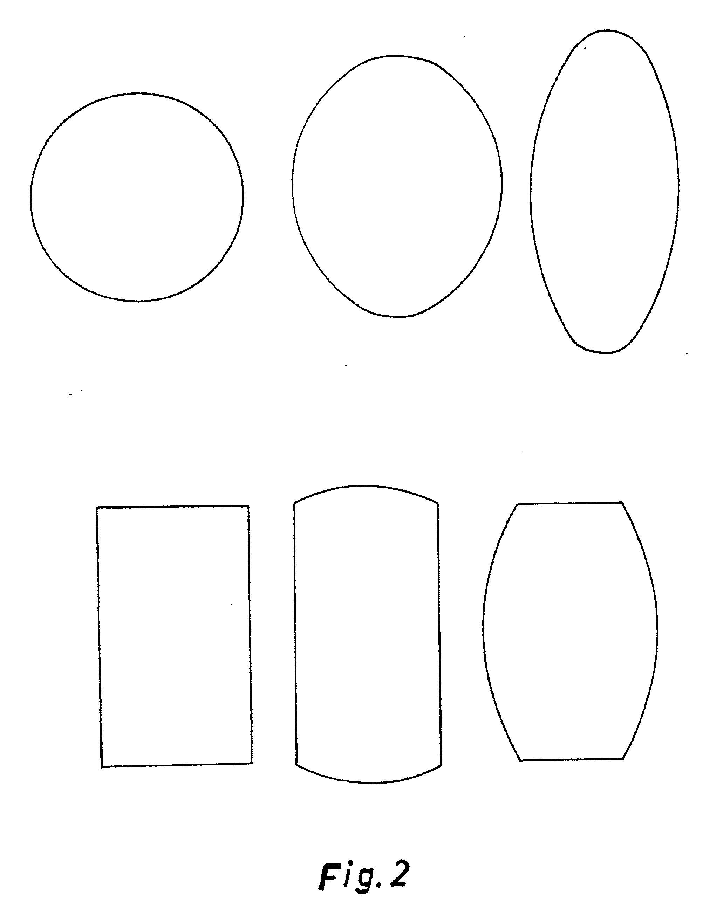

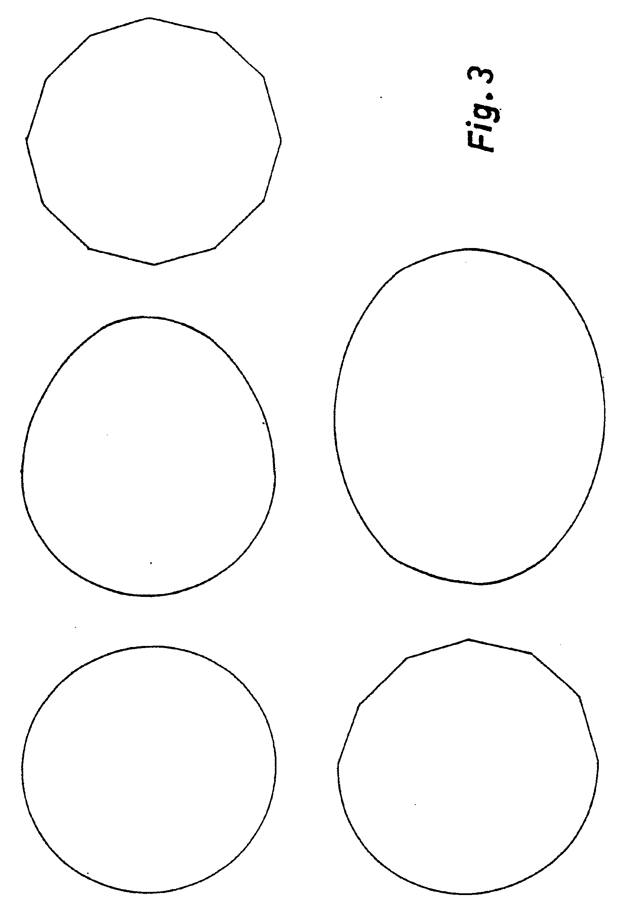

A few examples of the chamber designs in a device of the invention are illustrated in Figs. 1-3. Fig.1 illustrates perpendicular section planes a and b of the device intersecting along the axis 14 of the aerosol spray, and Figs. 2 and 3 show the corresponding examples of chamber outlines obtained by these planes. From the user's point of view it is preferable that the optimal configuration was found to be a distinctly laterally flattened shape (Fig. 2), whereby the device can be designed narrow and readily portable.

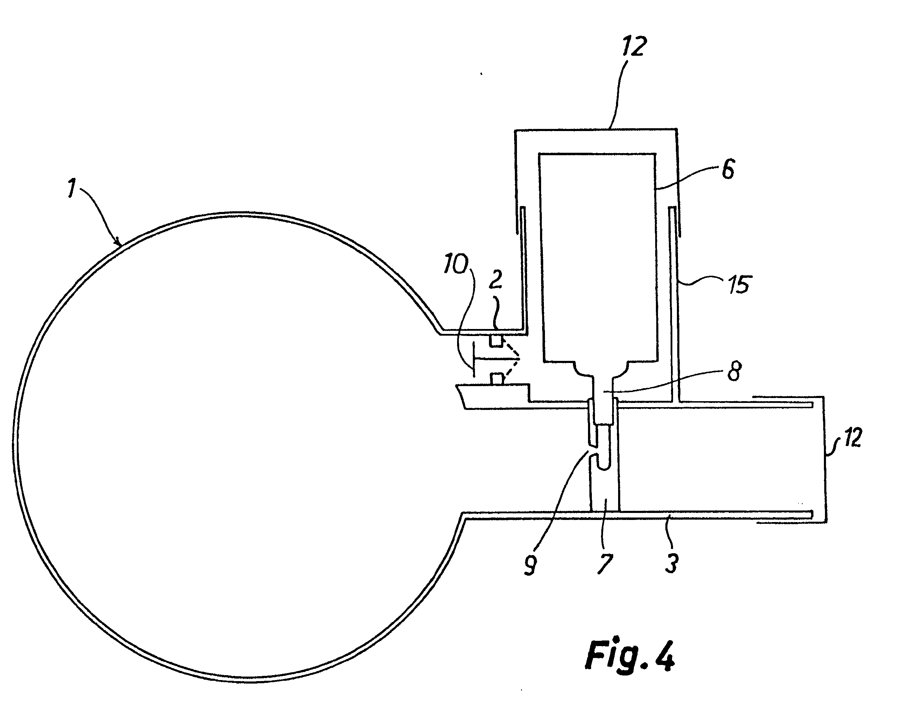

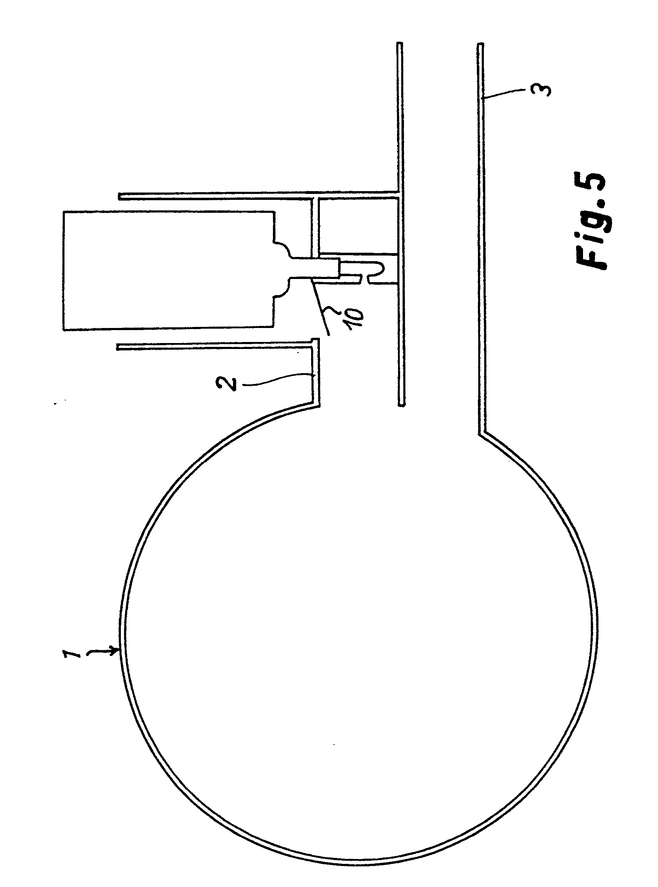

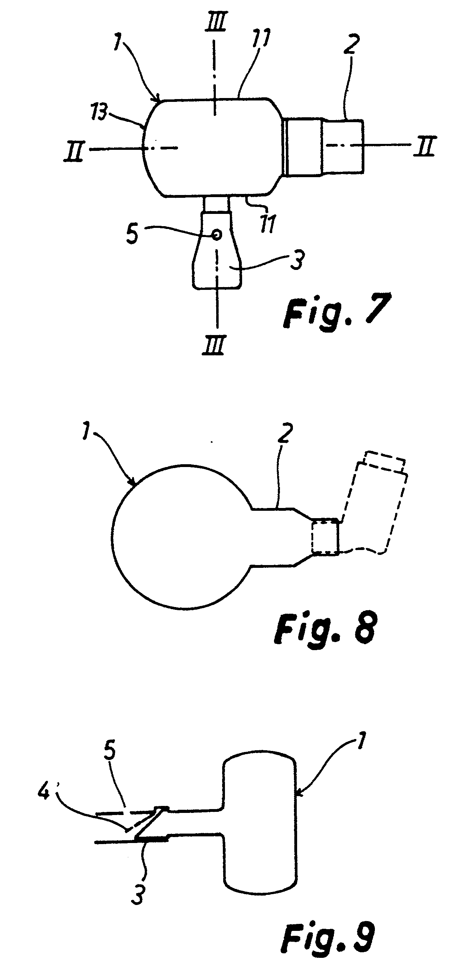

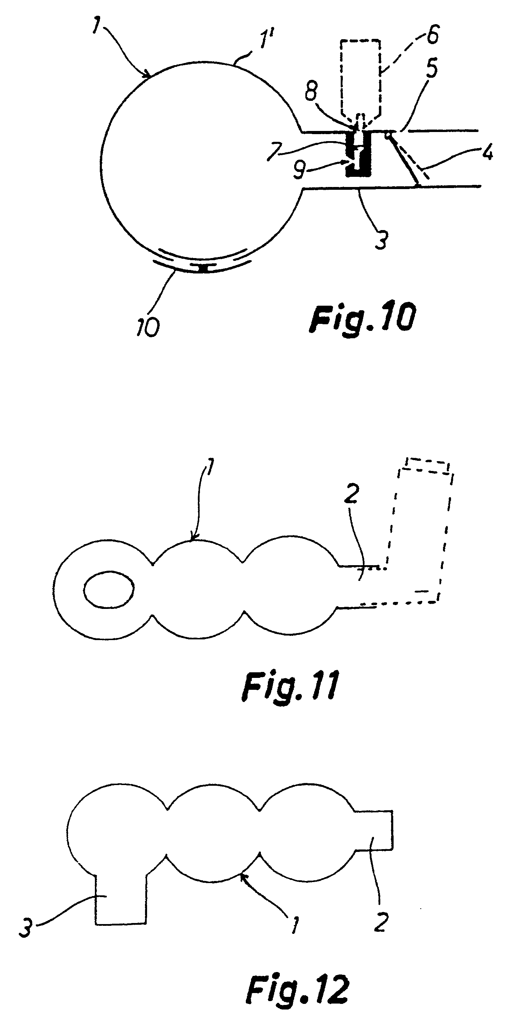

The structures of an aerosol container or a completed aerosol inhalation device as well as those of a supply duct, mouthpiece and an eventual one-way valve can be designed in several ways (Figs. 4-10). A medicine-containing aerosol container can be fitted to the device as an integral part thereof (Figs. 4, 5, 6 and 10), or the device can be provided with a separate inhalation aerosol container with its plastic casing (Figs. 7, 9). The supply or inlet duct and the mouthpiece can be made of one and the same tube (Figs. 4 and 10) or they can be separate components (Figs. 5, 6 and 7-9). In this case, the position of a mouthpiece must be experimentally optimized as its position has an effect on the operation of the device depending on the chamber configuration, volume and gas dosage.

Figs. 11 and 12 illustrate an embodiment consisting of three combined spherical chambers. It is obvious that the chambers can be of different shape and their number can be different from what is illustrated. The purpose of Figs. 11 and 12 is to demonstrate that the invention relates also to linked inhalation chambers.

As pointed out above, Figs. 1-3 illustrate a few basic embodiments of an inhalation chamber. Fig. 1 shows the cross-sectional planes along which the views of Figs. 2 and 3 are taken. In principle, the device comprises a spherical and flattened chamber 1 but, as shown in Fig. 3, the chamber configuration can nevertheless bo other than circular. The chamber outline can be circular, elliptical, polygonal or a combination of all these. The cross-section of an inhalation chamber shown in Fig. 2 can also vary. The shape can be an elliptical, polygonal, rectangular or various combinations thereof.

Fig. 4 illustrates an embodiment, wherein an inhalation chamber 1 of circular cross-section is provided with a mouthpiece 3 which also serves as an inlet duct for a medicament sprayed from an aerosol container 6. Thus, the duct of mouthpiece 3 is fitted with a socket 7 and its associated nozzle 9 for receiving an aerosol container valve stem 8. Thus, above said mouthpiece 3 is mounted a holder 15 for the aerosol container 6. Alongside said holder socket 15 is fitted a one-way valve 10 whereby air can flow into tie inhalation chamber 1 during inhalation.

Both holder 15 and mouthpiece 3 are also fitted with protective plugs 12 for preventing contamination.

The solution of Fig. 5 corresponds to that of Fig. 4 with a difference that mouthpiece 3 and inlet duct 2 are separated from each other.

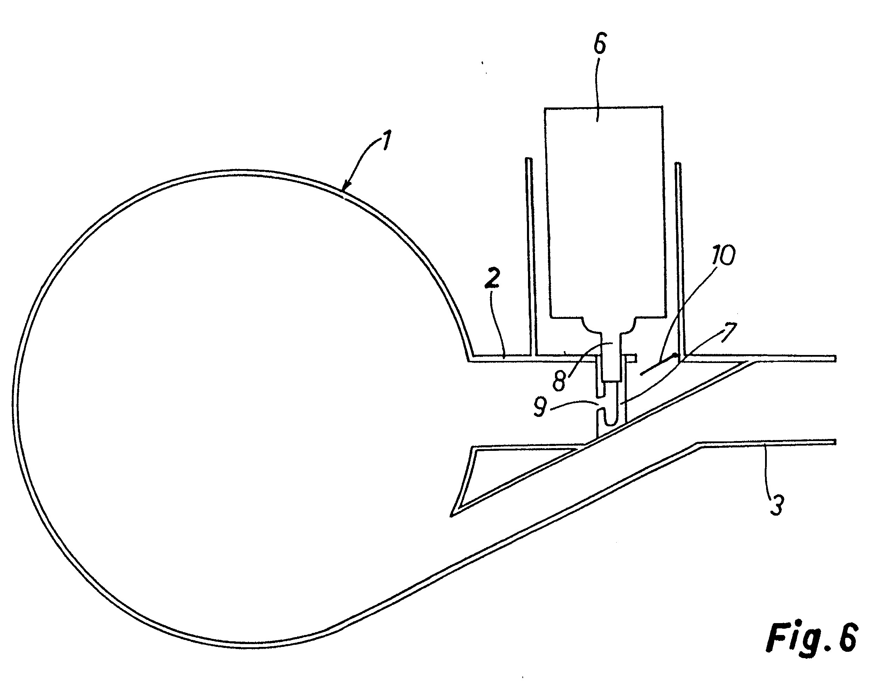

Fig. 6 discloses an embodiment which corresponds to that of Fig. 4 but also in this case mouthpiece 3 and inlet duct 2 are separated from each other. The embodiment of Fig. 6 differs from that of Fig. 5 in the way a one-way valve 10 is positioned and the duct of mouthpiece 3 is designed.

The device shown in Figs. 7-9 comprises a flattened spherical chamber 1. The opposite chamber walls 11 forming parts of chamber 1 lateral to the cross-section are flat or slightly convex surfaces, with a peripheral surface 13 therebetween. The cross-section of this surface 13 can be circular, eliptical or the like. The essential point is that a medicament spray is set in a turbulent or rotating motion in the chamber. Projecting from the wall 1' of the chamber 1 is an inlet or supply duct 2, designed so as to be readily fitted with a metering sprayer. Also projecting from ball like chamber 1 is a mouthpiece 3, fitted with a one-way valve 4. Said valve 4 only opens during inhalation. During exhalation, one-way valve 4 is shut off and air discharges through a hole 5. The imaginary axes II-II of inlet duct 2 and III-III of mouthpiece 3 form a 90° angle relative to each other. The device is operated as follows. The patient connects a metering sprayer to inlect duct 2, places mouthpiece 3 between his or her lips and triggers a dose of aerosol into chamber 1. The patient performs exhalation through mouthpiece 3, said one-way valve 4 being shutt off to prevent exhalation air from passing into chamber 1. Exhalation air discharges through a hole 5 in mouthpiece 3. The patient performs a deep, slow inhalation, whereby oneway valve 4 opens and the medicament particles floating in the chamber 1 are entrained and carried by inhalation air into the patient's lungs. Thus, the inhalation of a medicament is automatically synchronized with an inhalation step.

The device shown in Fig. 10 comprises a flattened, spherical chamber 1, out of the wall 1' of which extends a mouthpiece 3. This mouthpiece is provided with a socket 7, intended for an aerosol container 6 containing a medicament to be inhaled and fitted at the bottom thereof with a nozzle 9, the latter receiving a container valve stem 8 and opening towards the chamber. Said mouthpiece 3 is also fitted with a one-way valve 4 and a hole 5. The chamber bottom is provided with another one-way valve 10, wherethrough the air required for inhalation flows into the chamber. The device of Fig. 10 is operated the same way as that of Fig. 7 except that the device is now fitted with a mere aerosol can 6.

Figs. 11 and 12 illustrate a device the chamber 1 of which consists of three interlinked spherical sub-chambers. Figs. 11 shows the device in side view and Fig. 12 shows the same device in plan view.

The chamber 1 includes also an inlet duct 2, designed to be readily fitted with a metering sprayer (shown with dash-and-dot lines in Fig. 11). A mouthpiece 3 is mounted on the end of the chamber 1 opposite from inlet duct 2. The imaginary axes of mouthpiece 3 and inlet duct 2 form a 90° angle relative to each other.

The device is operated as follows. The patient connects a metering sprayer to inlet duct 2, performs a deep exhalation, places mouthpiece 3 between his or her lips, triggers aerosol and performs a deep inhalation.

The mouthpiece of such a device can be fitted with a one-way valve, if necessary, in which case there is no need for synchronization between aerosol release and inhalation.

As pointed out above, the final shape and linkage of interlinked inhalation chambers may vary within the scoped of the invention, regarding the shape, size and number of individual sub-chambers. Only as an example, Figs. 11 and 12 illustrates three interlinked balls.

## Claims

1. An inhalation device, comprising an inhalation chamber (1) fitted with an inlet duct (2) receiving a spray from a spray container (6) which contains a substance to be inhaled, as well as a mouthpiece (3), **characterized** in that the cross-section of inhalation chamber (1) in a plane defined by the longitudinal axis (14) of the aerosol spray substantially is in the shape of a circle, ellipse, polygon or a combination or interlinked arrays thereof and that the spraying into the chamber is in a non-radial direction in order to achieve a rotational movement of said spray and in that said chamber (1) may be flat at least in one of its lateral directions.

2. A device as set forth in claim 1, **characterized** in that the chamber is in the shape of a flattened ball or ellipsoid.

3. A device as set forth in claim 2, **characterized** in that the side walls (11) forming parts of the chamber lateral to said cross-section are flat or convex and a peripheral surface (13) therebetween forms a chamber outline substantially in the shape of a circle, ellipse, polygon or a combination thereof.

4. A device as set forth in claim 1, 2 or 3, **characterized** in that an inlet duct (2) and a mouthpiece (3) extend from the peripheral wall (1') of the chamber (1).

5. A device as set forth in claim 1, 2, 3 or 4, **characterized** in that said inlet duct (2) and mouthpiece (3) are an integral component in the peripheral wall (1') of said chamber (1).

6. A device as set forth in claim 5, **characterized** in that said inlet duct (2) and mouthpiece (3) constitute separate channels in a common component.

7. A device as set forth in claim 5, **characterized** in that the mouthpiece (3) is mounted perpendicular to the rotational plane of the aerosol spray.

8. A device as set forth in claim 5, **characterized** in that the mouthpiece (3) is fitted below the inlet duct (2).

9. A device as set forth in claims 4 and 5, **characterized** in that the inlet duct (2) is provided with a one-way valve (10).

10. A device as set forth in claims 4 and 5, **characterized** in that the mouthpiece (3) is provided with a one-way valve (4).

## Ansprüche

1. Inhalator mit einer Inhalatorkammer (1), die mit einem Einlaßkanal (2) versehen ist, in den ein Sprühstrahl aus einem Sprühmittelcontainer (6) mit einer zu inhalierenden Substanz eingebbar ist und der ein Mundstück (3) aufweist, dadurch gekennzeichnet, daß der Querschnitt der Inhalatorkammer (1) in einer durch die Längsachse (14) des Sprühstrahls definierten Ebene im wesentlichen die Gestalt eines Kreises, einer Ellipse, eines Polygons oder einer Kombination oder von miteinander verbundenen Anordnungen derselben aufweist, daß das Sprühen in die Kammer in einer nicht radialen Richtung erfolgt, um eine Drehbewegung des versprühten Mittels zu erzielen, und daß die Kammer (1) in wenigstens einer der seitlichen Richtungen flach sein kann.

2. Inhalator nach Anspruch 1, dadurch gekennzeichnet, daß die Kammer die Gestalt eines abgeflachten Balls oder Ellipsoids hat.

3. Inhalator nach Anspruch 2, dadurch gekennzeichnet, daß die zu beiden Seiten des Querschnitts gelegenen, Teile der Kammer bildenden Seitenwandungen (11) flach oder konvex sind und eine dazwischen gelegene Umfangsfläche (13) einen Umriß der Kammer im wesentlichen in der Gestalt eines Kreises, einer Ellipse, eines Polygons oder einer Kombination derselben bildet.

4. Inhalator nach Anspruch 1, 2 oder 3, dadurch gekennzeichnet, daß von der Umfangswandung (1') der Kammer (1) ein Einlaßkanal (2) und ein Mundstück (3) ausgehen.

5. Inhalator nach Anspruch 1, 2, 3 oder 4, dadurch gekennzeichnet, daß der Einlaßkanal (2) und das Mundstück (3) einheitliche Komponenten in der Umfangswandung (1') der Kammer bilden.

6. Inhalator nach Anspruch 5, dadurch gekennzeichnet, daß der Einlaßkanal (2) und das Mundstück (3) separate Kanäle in einer gemeinsamen Komponente bilden.

7. Inhalator nach Anspruch 5, dadurch gekennzeichnet, daß das Mundstück (3) senkrecht zur Rotationsebene des Aerosol-Sprühstrahls angebracht ist.

8. Inhalator nach Anspruch 5, dadurch gekennzeichnet, daß das Mundstück (3) unterhalb des Einlaßkanals (2) angeordnet ist.

9. Inhalator nach Anspruch 4 und 5, dadurch gekennzeichnet, daß der Einlaßkanal (2) mit einem Rückschlagventil (10) versehen ist.

10. Inhalator nach den Ansprüchen 4 und 5, dadurch gekennzeichnet, daß das Mundstück (3) mit einem Rückschlagventil (4) versehen ist.

caractérisé en ce qu'une conduite d'entrée (2) et un embout (3) partent de la paroi périphérique (1') de la chambre (1).

5. Inhalateur selon les revendications 1, 2, 3 ou 4, caractérisé en ce que la conduite d'entrée (2) et l'embout (3) font corps avec la paroi périphérique (1') de la chambre (1).

6. Inhalateur selon la revendication 5, caractérisé en ce que la conduite d'entrée (2) et l'embout (3) constituent des canaux distincts d'un composant commun.

7. Inhalateur selon la revendication 5, caractérisé en ce que l'embout (3) est monté perpendiculairement au plan de rotation de la pulvérisation d'aérosols.

8. Inhalateur selon la revendication 5, caractérisé en ce que l'embout (3) est emboité en-dessous de la conduite d'entrée (2).

9. Inhalateur selon les revendications 4 et 5, caractérisé en ce que la conduite d'entrée (2) comporte une valve à une alternance (10).

10. Inhalateur selon les revendications 4 et 5, caractérisé en ce que l'embout (3) comporte une valve à une alternance (4).

## Revendications

1. Inhalateur composé d'une chambre d'inhalation (1) munie d'une conduite d'entrée (2) recevant une pulvérisation d'un réservoir de pulvérisation (6) contenant une substance à inhaler ainsi qu'un embout (3), caractérisé en ce que la section transversale de la chambre d'inhalation (1) dans un plan défini par l'axe longitudinal (14) de la pulvérisation d'aérosols, a pratiquement la forme d'un cercle, d'une ellipse, d'un polygone ou d'une combinaison de ces figures et la pulvérisation dans la chambre se fait dans une direction non radiale pour engendrer un mouvement de rotation de la pulvérisation et en ce que la chambre (1) peut être plate au moins dans l'une de ses directions latérales.

2. Inhalateur selon la revendication 1, caractérisé en ce que la chambre a la forme d'une boule écrasée ou d'un ellipsoïde.

3. Inhalateur selon la revendication 2, caractérisé en ce que les parois latérales (11) faisant partie de la chambre à côté de la section, sont plates ou convexes et une surface périphérique (13) comprise entre ces parties latérales, forme une chambre dont le contour est essentiellement en forme de cercle, d'ellipse, de polygone ou d'une combinaison de ces figures.

4. Inhalateur selon les revendications 1, 2 ou 3,

*Fig.1*

Plane b

Plane a

1

14

EP 0 289 563 B1

**Fig. 2**

Fig. 3

Fig. 4

EP 0 289 563 B1

*Fig.5*

Fig. 6

Fig. 7

Fig. 8

Fig. 9

*Fig.10*

*Fig.11*

*Fig.12*